# EUROPEAN PATENT APPLICATION

(11) **EP 4 841 206 A1**
(43) Date of publication of application: **18.11.2026**
(21) Application number: 24916920.2
(22) Date of filing: 24.12.2024
(51) Int. Cl.: G01B 7/00

(54) **INFORMATION PROCESSING SYSTEM**

(30) Priority: 10.01.2024 JP 2024001849
(71) Applicant: OMRON Corporation, Kyoto-shi, Kyoto 600-8530 (JP)
(72) Inventor: NAMOSE Isamu, Kyoto-shi, Kyoto 600-8530 (JP)
(74) Representative: Marks & Clerk LLP
(86) International application number: PCT/JP2024/045728
(87) International publication number: WO 2025/150418

(57) **Abstract**

To provide an information processing system capable of suppressing the size and cost of an apparatus. An information processing system includes: an antenna that is a magnetic field generation device that generates a magnetic field; three coils that are respectively disposed on three axes orthogonal to each other and detect the magnetic field; a calculation unit that calculates a magnetic field strength based on a value of the magnetic field detected by each of the three coils; and a generation unit that generates posture information of a tag, which is an object including the three coils, based on a ratio of the value of the magnetic field detected by each of the three coils to the magnetic field strength calculated by the calculation unit.

## Description

### TECHNICAL FIELD

The present invention relates to an information processing system that generates posture information of an object.

### BACKGROUND OF INVENTION

Patent Document 1 below discloses a control system that estimates a region where a worker works in a production line of a factory or the like. The control system includes an acceleration sensor that detects acceleration data of each part of the worker and an angular velocity sensor that detects angular velocity data of each part of the worker. Then, the position information and the posture information of each part of the worker are calculated using the acceleration data and the angular velocity data acquired over time.

### CITATION LIST

### PATENT DOCUMENT

Patent Document 1: JP 2017-144489 A

### SUMMARY

### TECHNICAL PROBLEM

In the control system of Patent Document 1, it is necessary to provide two sensors, namely, an acceleration sensor and an angular velocity sensor, in order to calculate the position information and the posture information. This increases the size and cost of the apparatus.

In view of the above circumstances, an object of the present invention is to provide an information processing system capable of suppressing the size and cost of the apparatus.

### SOLUTION TO PROBLEM

An aspect of the present invention provides an information processing system including: a magnetic field generation device that generates a magnetic field; three coils that are respectively disposed on three axes orthogonal to each other and detect the magnetic field; a calculation unit that calculates a magnetic field strength based on a value of the magnetic field detected by each of the three coils; and a generation unit that generates posture information of an object including the three coils based on a ratio of the value of the magnetic field detected by each of the three coils to the magnetic field strength calculated by the calculation unit.

According to this aspect, the magnetic field generated from the magnetic field generation device is detected by the three coils disposed on the three axes orthogonal to each other, and the position information of the object can be obtained by using the magnetic field strength calculated based on the three detection values, and the posture information of the object including the three coils can be obtained by using the ratio of the three detection values to the magnetic field strength.

In the aspect described above, the generation unit may generate the posture information of the object by calculating an inclination angle of the object, which is an angle formed by an axial magnetic field component of each of the three axes and the object, using the ratio of the value of the magnetic field detected by each of the three coils to the magnetic field strength calculated by the calculation unit.

According to this aspect, the inclination angle of the object with respect to the axial magnetic field component of each of the three axes, which is calculated using the ratio of the three detection values to the magnetic field strength based on the three detection values, can be included in the posture information of the object including the three coils.

In the aspect described above, the generation unit may generate the posture information of the object by calculating a unit vector representing an inclination of the object with respect to an axial magnetic field component of each of the three axes using the ratio of the value of the magnetic field detected by each of the three coils to the magnetic field strength calculated by the calculation unit.

According to this aspect, the unit vector representing the inclination of the object with respect to the axial magnetic field component of each of the three axes, which is calculated using the ratio of the three detection values to the magnetic field strength based on the three detection values, can be included in the posture information of the object including the three coils.

In the aspect described above, at least three magnetic field generation devices may be provided.

According to this aspect, it is possible to calculate the position and the posture of the object using the positions of three or more points corresponding to the at least three magnetic field generation devices.

In the aspect described above, each of the magnetic field generation devices may generate a magnetic field continuously in order based on a periodic pattern for generating a magnetic field.

According to this aspect, it is possible to calculate the position and the posture of the object based on the magnetic fields generated continuously in order from the at least three magnetic field generation devices.

In the aspect described above, the magnetic field generation device may be an antenna including a coil that generates an electromagnetic wave with a low frequency (LF).

According to this aspect, for example, it is possible to improve the accuracy of distance measurement and the accuracy of measurement coordinates compared to UWB (Ultra Wide Band) or the like.

In the aspect described above, the object may be a tag including the three coils.

According to this aspect, it is possible to acquire the position information and the posture information of the tag including the three coils.

In the aspect described above, the tag may be attached to a target on which work operation is to be performed.

According to this aspect, it is possible to estimate, diagnose, and predict the operation of the target to which the tag is attached, based on the position information and the posture information of the tag.

### ADVANTAGEOUS EFFECTS OF INVENTION

According to the present invention, it is possible to provide an information processing system capable of suppressing the size and cost of the apparatus.

### BRIEF DESCRIPTION OF THE DRAWINGS

FIG. 1 is a schematic diagram illustrating the configuration of an information processing system according to an embodiment.
FIG. 2 is a block diagram illustrating the functional configuration of a tag illustrated in FIG. 1.
FIG. 3 is a schematic diagram for explaining the inclination angle of the tag in the X-axis and Y-axis directions.
FIG. 4 is a schematic diagram for explaining the inclination angle of the tag in the X-axis and Y-axis directions.
FIG. 5 is a schematic diagram for explaining the inclination angle of the tag in the X-axis and Y-axis directions.
FIG. 6 is a schematic diagram for explaining the inclination angle of the tag in the X-axis and Y-axis directions.

### DESCRIPTION OF EMBODIMENTS

A preferred embodiment of the present invention will be described with reference to the attached drawings. In the drawings, components denoted by the same reference signs have the same or similar configurations. However, the drawings are schematic, and specific dimensions and the like are to be determined in light of the following description. It goes without saying that different drawings also include parts with dimensional relationships and proportions different from one another.

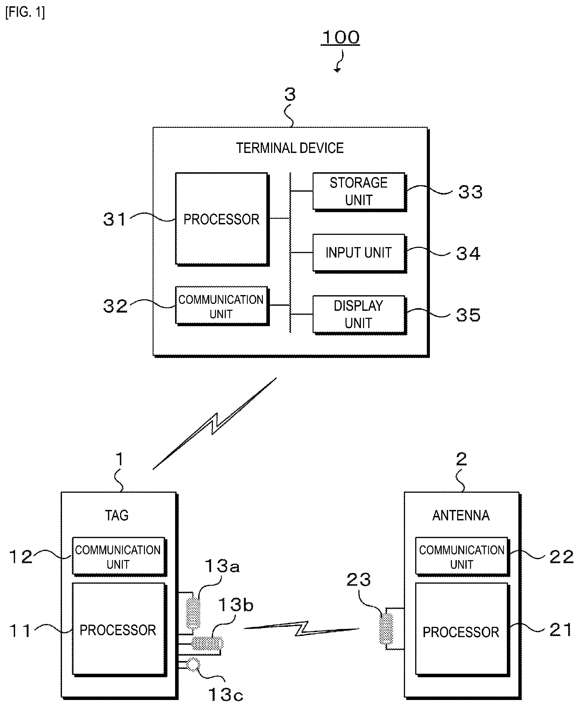

The configuration of an information processing system according to an embodiment will be described with reference to FIG. 1. An information processing system 100 illustrated in FIG. 1 includes, for example, a tag 1, an antenna 2, and a terminal device 3.

Before describing the constituent elements of the information processing system 100, an exemplary scene to which the present invention is applied will be described. The information processing system 100 according to the embodiment attaches the tag 1 to, for example, a hand, an arm, a hat, or the like of a worker who works in a predetermined process, detects a magnetic field generated from a coil 23 of the antenna 2 with three coils 13a, 13b, and 13c of the tag 1, and calculates the position and the posture of the tag 1 based on the detected values.

This makes it possible to collect data on the position and the posture of the tag 1 in time series in accordance with the movement of the tag 1. The terminal device 3 analyzes the data on the position and the posture of the tag 1, making it possible to estimate, diagnose, and predict the work operation, the stability of the work, and the like.

Here, the object to which the tag 1 is attached is not limited to the worker. For example, the tag 1 may be attached to a component, a jig or a tool, or the like that is used when the worker works. That is, it is preferable to attach the tag 1 to a target on which the work operation is to be performed.

The following describes the constituent elements of the information processing system 100 that implement such a scene.

The antenna 2 is a magnetic field generation device, and includes, for example, a processor 21 such as a MPU (micro processing unit) or a CPU (central processing unit), a communication unit 22 that enables wireless communication or wired communication, and a coil 23 that generates a magnetic field. The coil 23 preferably generates, for example, an electromagnetic wave with a low frequency (LF), and the antenna 2 preferably performs magnetic field communication in an LF band using the coil 23.

It is preferable that at least three antennas 2 are provided. The position and the posture of the tag 1 can be calculated by using the magnetic field communication between the tag 1 and at least three antennas 2. In this case, three coils 23 may be connected to one processor 21.

The processor 21 controls the coils 23 such that a current flows continuously in order through the coils 23 based on a periodic pattern for generating a magnetic field. This makes it possible to cause the coils 23 to sequentially and repeatedly generate a magnetic field. It is preferable that the periodic pattern and the current values are received from the terminal device 3 or the like via the communication unit 22 and stored in a memory.

The terminal device 3 is, for example, a personal computer, and includes a processor 31 such as a CPU or an MPU, a communication unit 32 that enables wireless communication or wired communication, a storage unit 33, an input unit 34, and a display unit 35.

The processor 31 executes a program stored in the storage unit 33 to implement various functions of the terminal device 3. The storage unit 33 is, for example, a computer-readable recording medium such as a disk drive or a semiconductor memory. The storage unit 33 stores the program for implementing the various functions of the terminal device 3, various data to be used by the program, and the like.

The input unit 34 is, for example, a keyboard, a touch panel, a mouse, a microphone, a camera, or the like. The display unit 35 is, for example, an organic EL display, a liquid crystal display, or the like.

The tag 1 is an electronic tag, and includes, for example, a processor 11 such as an MPU or a CPU, a communication unit 12 that enables wireless communication, and three coils 13a, 13b, and 13c. The three coils 13a, 13b, and 13c are disposed on three axes orthogonal to each other. Here, in the present embodiment, for example, the longitudinal direction of the coil 13a is defined as the X-axis direction, the longitudinal direction of the coil 13b is defined as the Y-axis direction, and the longitudinal direction of the coil 13c is defined as the Z-axis direction.

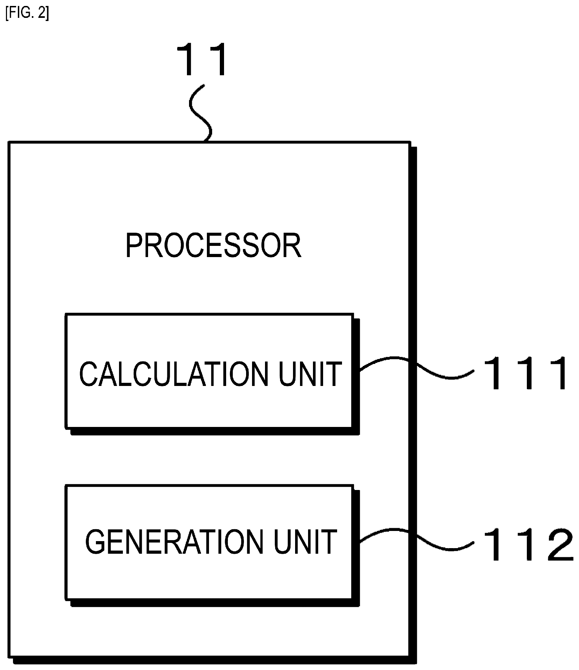

As illustrated in FIG. 2, the processor 11 of the tag 1 functions as, for example, a calculation unit 111 and a generation unit 112. Some or all of the functions of the calculation unit 111 and the generation unit 112 may be implemented by the processor 31 of the terminal device 3.

The calculation unit 111 calculates an RSSI (received signal strength indicator), which is a magnetic field strength, based on the value of the magnetic field detected by each of the three coils 13a, 13b, and 13c.

Here, the value of the magnetic field detected by the coil 13a indicating the X-axis direction is represented as RSSI_x as the X-axis component of the magnetic field strength RSSI, the value of the magnetic field detected by the coil 13b indicating the Y-axis direction is represented as RSSI_y as the Y-axis component of the magnetic field strength RSSI, and the value of the magnetic field detected by the coil 13c indicating the Z-axis direction is represented as RSSI_z as the Z-axis component of the magnetic field strength RSSI. In this case, the magnetic field strength RSSI can be calculated by combining the three components as in the following Equation (1), for example.

[Math 1] $RSSI = \sqrt{{\left(RSSI_x\right)}^{2} + {\left(RSSI_y\right)}^{2} + {\left(RSSI_z\right)}^{2}}$ (1)]

The distance between the antenna 2 and the tag 1 can be calculated based on the magnetic field strength RSSI calculated by Equation (1). Specifically, the distance between the antenna 2 and the tag 1 is calculated using the characteristic that the magnetic field strength RSSI attenuates with the cube of the distance.

The coordinates of the tag 1 can be calculated by providing three antennas 2 whose installation coordinates are known and calculating the distance between each of the antennas 2 and the tag 1. Specifically, the coordinates of the tag 1 are calculated as the intersection points of three circles, each of which has the installation coordinates of each antenna 2 as the center and the distance between each antenna 2 and the tag 1 as the radius. This makes it possible to obtain the position information of the tag 1.

The generation unit 112 generates the posture information of the tag 1 including the three coils 13a, 13b, and 13c based on the ratios of the values RSSI_x, RSSI_y, and RSSI_z of the magnetic fields detected by the three coils 13a, 13b, and 13c to the magnetic field strength RSSI calculated by the calculation unit 111.

Specifically, the generation unit 112 calculates the inclination angle of the tag 1, which is an angle formed by the axial magnetic field components of the three axes and the tag 1, using the magnetic field strength RSSI calculated by the calculation unit 111 and the values RSSI_x, RSSI_y, and RSSI_z of the magnetic fields detected by the three coils 13a, 13b, and 13c, and generates the posture information of the tag 1.

Here, the inclination angle of the tag 1 with respect to the X-axis direction is represented as angle_x, the inclination angle of the tag 1 with respect to the Y-axis direction is represented as angle_y, and the inclination angle of the tag 1 with respect to the Z-axis direction is represented as angle_z. In this case, the inclination angles angle_x, angle_y, and angle_z can be calculated by the following Equations (2), (3), and (4), respectively.$angle_x = {cos}^{- 1} \left(RSSI_x/RSSI\right)$ (2) $angle_y = {cos}^{- 1} \left(RSSI_y/RSSI\right)$ (3) $angle_z = {cos}^{- 1} \left(RSSI_z/RSSI\right)$ (4)

(RSSI_x/RSSI) in Equation (2), (RSSI_y/RSSI) in Equation (3), and (RSSI_z/RSSI) in Equation (4) are axial elements of a unit vector representing the inclination of the tag 1 with respect to the X-axis direction, the Y-axis direction, and the Z-axis direction, respectively. The unit vector (RSSI_x/RSSI, RSSI_y/RSSI, RSSI_z/RSSI) may be used as the posture information of the tag 1.

The inclination angles angle_x, angle_y, and angle_z of the tag 1 calculated by Equations (2), (3), and (4) are calculated for each antenna 2, and the calculated inclination angles are used as the posture information of the tag 1. The inclination angles angle_x, angle_y, and angle_z of the tag 1 are angles indicating how much the tag 1 is inclined from a reference angle (0 degrees) at which the tag 1 is directed to the antenna 2.

The inclination angles angle_x, angle_y, and angle_z of the tag 1 are angles in a coordinate system (hereinafter, also referred to as a "world coordinate system") based on a state in which the tag 1 is directed to the antenna 2.

In contrast, the inclination of the tag 1 can be represented by angles in a coordinate system (hereinafter, also referred to as a "global coordinate system") representing the installation coordinates of the antenna. The angles in the global coordinate system can be calculated by affine transformation of the angles in the world coordinate system. In other words, the angles in the global coordinate system can be calculated by transforming the angles in the world coordinate system using an affine transformation matrix. The angles in the global coordinate system may be used as the posture information of the tag 1.

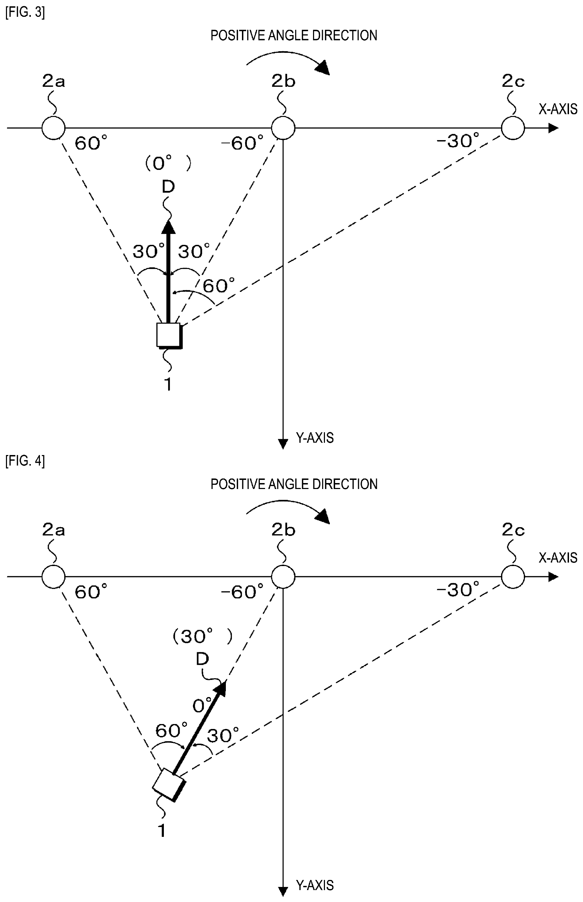

The inclination angle of the tag 1 will be specifically described with reference to FIGS. 3 to 6. For convenience of description, the inclination angles in the X-axis and Y-axis directions are illustrated in FIGS. 3 to 6. The same description as the description for the X-axis and Y-axis directions can be made for the other directions, namely the Y-axis and Z-axis directions and the Z-axis and X-axis directions, and thus such descriptions will be omitted.

In FIGS. 3 to 6, it is assumed that three antennas 2a, 2b, and 2c are installed on the X-axis and the installation coordinates of such antennas are known. By way of example, the three antennas 2a, 2b, and 2c can be disposed on a workbench on which the worker works, and the tag 1 can be attached to a hand of the worker.

FIG. 3 is a schematic diagram based on the premise that a direction (posture) D of the tag 1 is a direction perpendicular to the X-axis and parallel to the Y-axis. The direction D of the tag 1 in this case is defined as the direction at 0 degrees in the global coordinate system.

Here, since the directions of the coils 13a, 13b, and 13c cannot be specified, it cannot be determined whether the angles calculated based on the magnetic fields detected by the coils are positive or negative. Therefore, the angles in the world coordinate system calculated based on the magnetic fields detected by the coils are calculated as absolute values.

In FIG. 3, the inclination angle of the tag 1 with respect to the antenna 2a is calculated as 30 degrees, the inclination angle of the tag 1 with respect to the antenna 2b is calculated as 30 degrees, and the inclination angle of the tag 1 with respect to the antenna 2c is calculated as 60 degrees. That is, the inclination angle of the tag 1 with respect to the antenna 2a and the inclination angle of the tag 1 with respect to the antenna 2b are both calculated as 30 degrees.

Here, since the installation coordinates of the antennas 2a, 2b, and 2c are known and the arrangement in the X-axis direction is also known, the inclination angle of the tag 1 with respect to the antenna 2a should be smaller than the inclination angle of the tag 1 with respect to the antenna 2b. Therefore, it can be determined that the inclination angle of the tag 1 with respect to the antenna 2a is not 30 degrees but -30 degrees.

In this case, the information that the inclination angle of the tag 1 with respect to the antenna 2a is -30 degrees, the inclination angle of the tag 1 with respect to the antenna 2b is 30 degrees, and the inclination angle of the tag 1 with respect to the antenna 2c is 60 degrees in the X-axis and Y-axis directions is included in the posture information of the tag 1. The inclination angles with respect to the antennas 2a, 2b, and 2c for the other directions, namely the Y-axis and Z-axis directions and the Z-axis and X-axis directions, can be calculated in the same manner as for the X-axis and Y-axis directions.

FIG. 4 is a schematic diagram based on the premise that the direction D of the tag 1 is a direction rotated clockwise by 30 degrees from the direction in FIG. 3. The direction D of the tag 1 in this case is defined as the direction at 30 degrees in the global coordinate system.

In FIG. 4, the inclination angle of the tag 1 with respect to the antenna 2a is calculated as 60 degrees, the inclination angle of the tag 1 with respect to the antenna 2b is calculated as 0 degrees, and the inclination angle of the tag 1 with respect to the antenna 2c is calculated as 30 degrees. That is, the inclination angle of the tag 1 with respect to the antenna 2a is calculated as a larger value than the inclination angle of the tag 1 with respect to the antenna 2b.

Here, since the installation coordinates of the antennas 2a, 2b, and 2c are known and the arrangement in the X-axis direction is also known, the inclination angle of the tag 1 with respect to the antenna 2a should be smaller than the inclination angle of the tag 1 with respect to the antenna 2b. Therefore, it can be determined that the inclination angle of the tag 1 with respect to the antenna 2a is not 60 degrees but -60 degrees.

In this case, the information that the inclination angle of the tag 1 with respect to the antenna 2a is -60 degrees, the inclination angle of the tag 1 with respect to the antenna 2b is 0 degrees, and the inclination angle of the tag 1 with respect to the antenna 2c is 30 degrees in the X-axis and Y-axis directions is included in the posture information of the tag 1. The inclination angles with respect to the antennas 2a, 2b, and 2c for the other directions, namely the Y-axis and Z-axis directions and the Z-axis and X-axis directions, can be calculated in the same manner as for the X-axis and Y-axis directions.

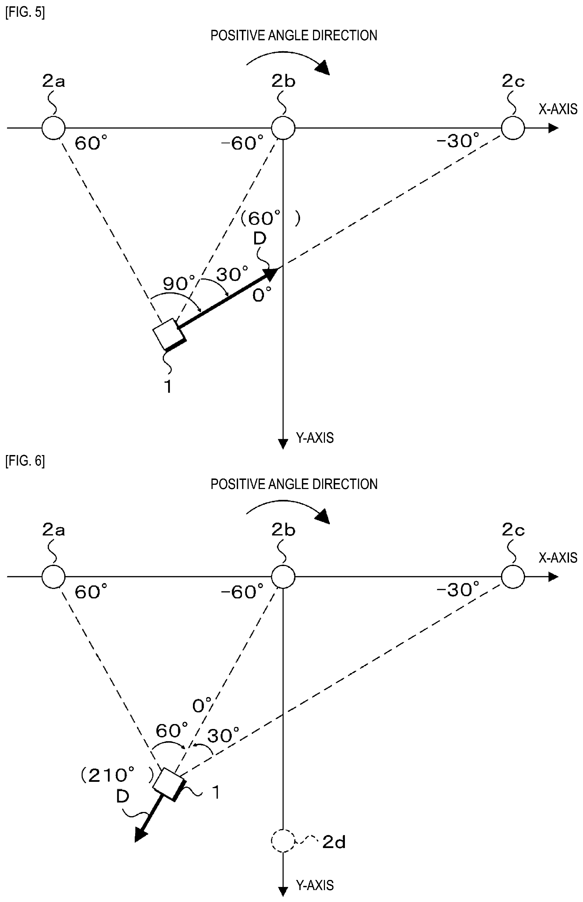

FIG. 5 is a schematic diagram based on the premise that the direction D of the tag 1 is a direction rotated clockwise by 60 degrees from the direction in FIG. 3. The direction D of the tag 1 in this case is defined as the direction at 60 degrees in the global coordinate system.

In FIG. 5, the inclination angle of the tag 1 with respect to the antenna 2a is calculated as 90 degrees, the inclination angle of the tag 1 with respect to the antenna 2b is calculated as 30 degrees, and the inclination angle of the tag 1 with respect to the antenna 2c is calculated as 0 degrees. That is, the inclination angle of the tag 1 with respect to the antenna 2a is calculated as a larger value than the inclination angle of the tag 1 with respect to the antenna 2b, and the inclination angle of the tag 1 with respect to the antenna 2b is calculated as a larger value than the inclination angle of the tag 1 with respect to the antenna 2c.

Here, since the installation coordinates of the antennas 2a, 2b, and 2c are known and the arrangement in the X-axis direction is also known, the inclination angle of the tag 1 with respect to the antenna 2a should be smaller than the inclination angle of the tag 1 with respect to the antenna 2b, and the inclination angle of the tag 1 with respect to the antenna 2b should be smaller than the inclination angle of the tag 1 with respect to the antenna 2c. Therefore, it can be determined that the inclination angle of the tag 1 with respect to the antenna 2a is not 90 degrees but -90 degrees, and it can be determined that the inclination angle of the tag 1 with respect to the antenna 2b is not 30 degrees but -30 degrees.

In this case, the information that the inclination angle of the tag 1 with respect to the antenna 2a is -90 degrees, the inclination angle of the tag 1 with respect to the antenna 2b is -30 degrees, and the inclination angle of the tag 1 with respect to the antenna 2c is 0 degrees in the X-axis and Y-axis directions is included in the posture information of the tag 1. The inclination angles with respect to the antennas 2a, 2b, and 2c for the other directions, namely the Y-axis and Z-axis directions and the Z-axis and X-axis directions, can be calculated in the same manner as for the X-axis and Y-axis directions.

FIG. 6 is a schematic diagram based on the premise that the direction D of the tag 1 is a direction rotated clockwise by 210 degrees from the direction in FIG. 3. The direction D of the tag 1 in this case is defined as the direction at 210 degrees in the global coordinate system.

In FIG. 6, the inclination angle of the tag 1 with respect to the antenna 2a is calculated as 60 degrees, the inclination angle of the tag 1 with respect to the antenna 2b is calculated as 0 degrees, and the inclination angle of the tag 1 with respect to the antenna 2c is calculated as 30 degrees. That is, the same inclination angles as those in the state in FIG. 4 described above are calculated. This is because the directions of the coils 13a, 13b, and 13c cannot be specified, and it cannot be determined whether the angles calculated based on the magnetic fields detected by the coils are positive or negative.

In this way, when the direction D of the tag 1 is reversed from the direction facing each of the antennas 2a, 2b, and 2c, it is preferable to add another antenna 2d at a coordinate position larger than the Y coordinate of the tag 1 in the positive direction of the Y-axis, for example. By adding the antenna 2d in this manner, the direction of the tag 1 in the Y-axis direction can be determined.

This makes it possible to determine whether the worker to whom the tag 1 is attached is working while facing the workbench on which the antennas 2a, 2b, and 2c are disposed or is working with his/her back facing the workbench.

In FIG. 6, by adding the antenna 2d, it can be determined that the inclination angle of the tag 1 with respect to the antenna 2a is 120 degrees, the inclination angle of the tag 1 with respect to the antenna 2b is 180 degrees, and the inclination angle of the tag 1 with respect to the antenna 2c is 210 degrees.

In this case, the information that the inclination angle of the tag 1 with respect to the antenna 2a is 120 degrees, the inclination angle of the tag 1 with respect to the antenna 2b is 180 degrees, and the inclination angle of the tag 1 with respect to the antenna 2c is 210 degrees in the X-axis and Y-axis directions is included in the posture information of the tag 1. The inclination angles with respect to the antennas 2a, 2b, and 2c for the other directions, namely the Y-axis and Z-axis directions and the Z-axis and X-axis directions, can be calculated in the same manner as for the X-axis and Y-axis directions.

Here, for example, when analyzing operation using AI, it is only necessary to grasp the transition of the operation, and thus information as to whether the angles calculated based on the magnetic fields detected by the coils are positive or negative is not essential. That is, information as to whether such angles are positive or negative can be omitted. Therefore, in such a case, it is sufficient to dispose three antennas 2a, 2b, and 2c, and thus it is not necessary to add another antenna 2d.

As described above, according to the information processing system 100 according to the embodiment, the magnetic fields generated from the three antennas 2a, 2b, and 2c are detected by the three coils 13a, 13b, and 13c disposed on the three axes orthogonal to each other, and the position information of the tag 1 can be obtained by using the magnetic field strength RSSI calculated based on the three detection values RSSI_x, RSSI_y, and RSSI_z, and the posture information of the tag 1 including the three coils 13a, 13b, and 13c can be obtained by using the ratios of the three detection values RSSI_x, RSSI_y, and RSSI_z to the magnetic field strength RSSI.

Therefore, for example, when the tag 1 is attached to a hand of the worker, the direction (posture) of the hand can be acquired at the same time as the movement of the hand. This makes it possible to acquire information indicating that the hand has moved in a wobbling manner, or that the arm has been stretched with the wrist placed vertically or horizontally. Based on such information, it is also possible to present a difference between the operation of an experienced worker and the operation of a new worker.

Thus, the information processing system 100 according to the embodiment can omit the acceleration sensor and the angular velocity sensor for calculating the position information and the posture information, and thus can reduce the size and cost of the apparatus.

The embodiment described above is merely an example of the present invention in all respects. It goes without saying that various improvements and variations may be made without departing from the scope of the present invention. That is, in implementing the present invention, specific configurations according to the embodiment may be adopted as appropriate. The above-described embodiment is provided for facilitating understanding of the present invention, and is not to provide limited interpretation of the present invention.

### [Appendices]

Aspects of the present embodiment include the following disclosures.

### (Appendix 1)

An information processing system (100) including:
a magnetic field generation device (2) that generates a magnetic field;
three coils (13a, 13b, 13c) that are respectively disposed on three axes orthogonal to each other and detect the magnetic field;
a calculation unit (111) that calculates a magnetic field strength based on a value of the magnetic field detected by each of the three coils (13a, 13b, 13c); and
a generation unit (112) that generates posture information of an object (1) including the three coils (13a, 13b, 13c) based on a ratio of the value of the magnetic field detected by each of the three coils (13a, 13b, 13c) to the magnetic field strength calculated by the calculation unit (111).

### (Appendix 2)

The information processing system (100) according to Appendix 1, in which the generation unit (112) generates the posture information of the object (1) by calculating an inclination angle of the object (1), which is an angle formed by an axial magnetic field component of each of the three axes and the object, using the ratio of the value of the magnetic field detected by each of the three coils (13a, 13b, 13c) to the magnetic field strength calculated by the calculation unit (111).

### (Appendix 3)

The information processing system (100) according to Appendix 1 or 2, in which the generation unit (112) generates the posture information of the object (1) by calculating a unit vector representing an inclination of the object (1) with respect to an axial magnetic field component of each of the three axes using the ratio of the value of the magnetic field detected by each of the three coils (13a, 13b, 13c) to the magnetic field strength calculated by the calculation unit (111).

### (Appendix 4)

The information processing system (100) according to any one of Appendices 1 to 3, in which
at least three magnetic field generation devices (2) are provided.

### (Appendix 5)

The information processing system (100) according to Appendix 4, in which each of the magnetic field generation devices (2) generates a magnetic field continuously in order based on a periodic pattern for generating a magnetic field.

### (Appendix 6)

The information processing system (100) according to any one of Appendices 1 to 5, in which
the magnetic field generation device (2) is an antenna (2) including a coil that generates an electromagnetic wave with a low frequency (LF).

### (Appendix 7)

The information processing system (100) according to any one of Appendices 1 to 6, in which
the object (1) is a tag (1) including the three coils (13a, 13b, 13c).

### (Appendix 8)

The information processing system (100) according to Appendix 7, in which the tag (1) is attached to a target on which work operation is to be performed.

### REFERENCE SIGNS

1 tag (object), 2a, 2b, 2c, 2d antenna (magnetic field generation device), 3 terminal device, 11 processor, 12 communication unit, 13a, 13b, 13c coil, 21 processor, 22 communication unit, 23 coil, 31 processor, 32 communication unit, 33 storage unit, 34 input unit, 35 display unit, 100 information processing system, 111 calculation unit, 112 generation unit

## Claims

1. An information processing system (100) comprising:
a magnetic field generation device (2) that generates a magnetic field;
three coils (13a, 13b, 13c) that are respectively disposed on three axes orthogonal to each other and detect the magnetic field;
a calculation unit (111) that calculates a magnetic field strength based on a value of the magnetic field detected by each of the three coils (13a, 13b, 13c); and
a generation unit (112) that generates posture information of an object (1) including the three coils (13a, 13b, 13c) based on a ratio of the value of the magnetic field detected by each of the three coils (13a, 13b, 13c) to the magnetic field strength calculated by the calculation unit (111).

2. The information processing system (100) according to claim 1, wherein
the generation unit (112) generates the posture information of the object (1) by calculating an inclination angle of the object (1), which is an angle formed by an axial magnetic field component of each of the three axes and the object (1), using the ratio of the value of the magnetic field detected by each of the three coils (13a, 13b, 13c) to the magnetic field strength calculated by the calculation unit (111).

3. The information processing system (100) according to claim 1, wherein
the generation unit (112) generates the posture information of the object (1) by calculating a unit vector representing an inclination of the object (1) with respect to an axial magnetic field component of each of the three axes using the ratio of the value of the magnetic field detected by each of the three coils (13a, 13b, 13c) to the magnetic field strength calculated by the calculation unit (111).

4. The information processing system (100) according to claim 1, wherein
at least three magnetic field generation devices (2) are provided.

5. The information processing system (100) according to claim 4, wherein
each of the magnetic field generation devices (2) generates a magnetic field continuously in order based on a periodic pattern for generating a magnetic field.

6. The information processing system (100) according to claim 1, wherein
the magnetic field generation device (2) is an antenna (2) including a coil that generates an electromagnetic wave with a low frequency (LF).

7. The information processing system (100) according to claim 1, wherein
the object (1) is a tag (1) including the three coils (13a, 13b, 13c).

8. The information processing system (100) according to claim 7, wherein
the tag (1) is attached to a target on which work operation is to be performed.
